Europäisches Patentamt

European Patent Office

Office européen des brevets

(11) Veröffentlichungsnummer : **0 087 062**
**B1**

(12) **EUROPÄISCHE PATENTSCHRIFT**

(45) Veröffentlichungstag der Patentschrift :
11.12.85

(51) Int. Cl.⁴ : **A 61 K 9/06, A 61 K 31/19**

(21) Anmeldenummer : 83101214.1

(22) Anmeldetag : 09.02.83

(54) Äusserlich anwendbares, Ibuprofen enthaltendes Arzneimittel und Verfahren zu seiner Herstellung.

(30) Priorität : 16.02.82 DE 3205504

(43) Veröffentlichungstag der Anmeldung :
31.08.83 Patentblatt 83/35

(45) Bekanntmachung des Hinweises auf die Patenterteilung : 11.12.85 Patentblatt 85/50

(84) Benannte Vertragsstaaten :
AT BE CH DE FR GB IT LI LU NL SE

(56) Entgegenhaltungen :
EP-A- 0 063 870
CHEMICAL ABSTRACTS, Band 95, Nr. 2, 13. Juli 1981, Seite 356, Nr. 12662s, Columbus, Ohio, US G. BRAMANTI u.a.: "Release of drugs in vitro from different dermatological preparations"
PATENTS ABSTRACTS OF JAPAN, Band 6, Nr. 7 (C-87)885, 16. Januar 1982
PATENTS ABSTRACTS OF JAPAN, Band 6, Nr. 250 (C-139)1128, 9. Dezember 1982

(73) Patentinhaber : Dolorgiet GmbH & Co. KG
Otto-von-Guericke-Str. 1
5205 St. Augustin 3 (DE)

(72) Erfinder : Gruber, Klaus, Dr.
Im Bonnefeld 6
D-5480 Remagen 2 (DE)
Erfinder : Löhner, Manfred
Blücherstrasse 47
D-5300 Bonn 1 (DE)
Erfinder : Posselt, Klaus, Dr.
Rodderbergstrasse 62
D-5300 Bonn 2 (DE)
Erfinder : Wagener, Hans Henrich, Prof. Dr. med.
Breslauerstrasse 76
D-5309 Meckenheim (DE)

(74) Vertreter : Werner, Hans-Karsten, Dr. et al
Deichmannhaus am Hauptbahnhof
D-5000 Köln 1 (DE)

# 0 087 062

**Beschreibung**

Ibuprofen, 2-(4-Isobutylphenyl)-propionsäure, (The Merck Index 1976, 4796, 9th Ed., Merck & Co. Inc.) ist ein bekannter Arzneistoff, der in breitem Rahmen als Antirheumatikum/Antiphlogistikum und Analgetikum vorwiegend bei degenerativen und entzündlichen Gelenkerkrankungen und Weichteilrheumatismus eingesetzt wird.

Die Verabreichung von Ibuprofen erfolgt vornehmlich in Form von Dragées und Suppositorien. Die orale und, bei systemisch wirksamen Arzneistoffen wie Ibuprofen, auch rektale Anwendung hat jedoch nachteilige Wirkungen, wie gastrointestinale Beschwerden, Schwindel, Übelkeit, Kopfschmerzen. Bei Patienten mit Ulcus ventriculi und duodeni ist eine orale Behandlung mit Ibuprofen ausgeschlossen.

Bei topischer Anwendung erreicht ein Arzneistoff unmittelbar das Zielorgan unter Umgehung des bei oraler oder rektaler Verabreichung unumgänglichen First-pass-Effektes, d. h. der ersten Verstoffwechselung in der Leber. Dadurch ist es möglich, die bei oraler und rektaler Anwendung auftretenden Nachteile zu vermindern.

Zur lokalen Anwendung muß ein Wirkstoff z. B. in eine Creme, eine Salbe oder ein Gel eingearbeitet werden. Eine Ibuprofencreme kann nach den üblichen Methoden, wie sie für die Cremeherstellung gelten, nicht hergestellt werden, weil Ibuprofen im Wasser und üblichen Medien nur wenig löslich ist.

Offensichtlich wurden Versuche, eine solche Creme herzustellen bereits unternommen. So zeigte die mikroskopische Untersuchung einer im Handel befindlichen 10 %igen Ibuprofencreme (L'Informatore Farmaceutico 1981, A-107), daß der Wirkstoff hier weitgehend ungelöst in Form feiner nadelförmiger Kristalle vorliegt. Diese Creme hat jedoch bisher keine breite Anwendung gefunden. Der Grund kann darin gesehen werden, daß ein lokal anwendbares Arzneimittel nur gut und rasch wirken kann, wenn der Wirkstoff entsprechend durch die Haut penetriert. Das jedoch setzt einen gelösten Wirkstoff voraus.

Die Erfindung hat sich die Aufgabe gestellt, ein äußerlich anwendbares Arzneimittel zur Verfügung zu stellen, welches eine wirksame Menge Ibuprofen in einem Träger enthält. Diese Aufgabe wird dadurchgelöst, daß man Ibuprofen in einer Mischung aus mittelkettigen Triglyceriden, Glycerinmonostearat-Polyoxy-äthylenstearat-Gemisch und Polyoxyethylen-Fettsäureester löst. Die Lösung kann in an sich bekannter Weise durch weitere Zusätze in eine Creme, eine Salbe oder ein Gel weiterverarbeitet werden. Vorzugsweise stellt man aus dieser Lösung eine Creme her. Der wässrigen Phase der Creme können Zusätze wie 1,2-Propandiol (2 bis 10 Gew.teile), Konservierungsmittel wie 4-Hydroxy-Benzoesäuremethylester-Natrium (0,1 bis 1 Gew.teile) und Verdickungsmittel wie Xanthan-Gummi (0,1 bis 1 Gew.teile) zugesetzt werden.

Gegenstand der vorliegenden Anmeldung ist somit ein äußerlich anwendbares Arzneimittel, das eine wirksame Menge Ibuprofen in einem Träger enthält, das dadurch gekennzeichnet ist, daß es 2 bis 12 Gewichtsteile Ibuprofen enthält, gelöst in einer Mischung aus 30 bis 50 Gewichtsteilen mittelkettiger Triglyceride, 4 bis 10 Gewichtsteilen Glycerinmonostearat-Polyoxyethylenstearatgemisch und 2 bis 10 Gewichtsteilen Polyoxyethylen-Fettsäureester sowie ein Verfahren zur Herstellung solcher Arzneimittel.

Als wirksame Menge Ibuprofen kommen 2 bis 12 Gewichtsteile, vorzugsweise 4 bis 8 Gewichtsteile infrage. Eine gute Lösung und gute Resorption wird beobachtet, wenn Ibuprofen gelöst ist in oben genannten 3 Komponenten in den dort angegebenen Mengen.

Die Triglyceride enthalten vorzugsweise $C_6$-$C_{12}$-Carbonsäure, die Polyoxyethylen-Fettsäureester $C_{12}$-$C_{18}$-Carbonsäuren. Die Ethylenoxideinheiten des Polyoxyethylrestes variieren zwischen 6 und 100. In der erfindungsgemäßen Creme können übliche Zusatzstoffe, wie oberflächenaktive Mittel, Lösungsmittel, Bindemittel, Konservierungsstoffe und Parfüme, enthalten sein.

Die Herstellung und Zusammensetzung einer erfindungsgemäßen Creme wird in den nachfolgenden Beispielen näher erläutert :

Folgende Komponenten ergeben typische Rezepturen für Cremes :

| | |
|---|---|
| Ibuprofen | 4,0- 5,5 g |
| Mittelkettige Triglyceride (DAB 8) | 35,0- 42,0 g |
| Glycerinmonostearat-Polyoxyethylenstearat-Gemisch | 6,0- 8,0 g |
| Polyoxyethylen-Fettsäureester | 4,0- 6,0 g |
| 1,2-Propandiol | 4,0- 6,0 g |
| 4-Hydroxybenzoesäuremethylester-Natrium | 0,1- 0,4 g |
| Xanthan-Gummi | 0,2- 0,5 g |
| Wasser jeweils ad | 100,0 g |

Die Herstellung der Creme erfolgt in an sich bekannter Weise, wie nachstehendes Beispiel veranschaulicht :

Die ölige Phase, bestehend aus mittelkettigen Triglyceriden, Glycerinmonostearat-Polyoxyethylenstearat-Gemisch sowie Polyoxyethylen-Fettsäureester, wird auf 60-65 °C erwärmt. Darin löst man die entsprechende Menge Ibuprofen unter Rühren.

Die wässrige Phase, bestehend aus 1,2-Propandiol, 4-Hydroxybenzoesäuremethylester-Natrium, Xanthan-Gummi und der entsprechenden Menge Wasser wird ebenfalls auf 60-65 °C erwärmt.

Die wässrige Phase wird portionsweise unter Rühren der öligen Phase zugesetzt. Anschließend wird der Ansatz bis zur Abkühlung auf Raumtemperatur gleichmäßig gerührt.

Die zu applizierende Menge und Häufigkeit hängt im wesentlichen vom Grad und der Art der zu behandelnden Krankheit ab. Vorzugsweise wird das schmerzhafte Gebiet 3-6 mal täglich mit einem 5-10 cm langen Strang Creme (entsprechend 200-400 mg Ibuprofen) eingerieben.

Als Indikationen kommen in Betracht degenerative und entzündliche Gelenkerkrankungen, Weichteilrheumatismus, Lumbago, Myogelosen und traumatische Zustände nach Sportverletzungen und Unfällen. Bei Anwendung der erfindungsgemäßen Zubereitungsform kommt man mit geringeren Mengen des Wirkstoffes Ibuprofen aus als bei Anwendung der handelsüblichen Creme.

Wirkung am Carrageenin-induziertem Pfotenödem der Ratte

Männliche Sprague-Dawley-Ratten von jeweils 80-100 g KG wurden am Rücken kahlgeschoren. Durch subplantare Injektion 0,1 ml einer 1 %igen Carrageenin-Lösung in die linke Hinterpfote wurde das Ödem provoziert. 30 Minuten nach der Injektion wurde am kahlgeschorenen Rücken der Tiere die Menge Creme eingerieben, die 100 mg/kg KG Ibuprofen entspricht. Die eingeriebene Fläche betrug jeweils 15-20 cm². Unmittelbar nach der Injektion und dann im Abstand von je 15 Stunden nach der Einreibung wurden die Pfotenvolumina bestimmt. Der Inhibierungsgrad des Ödems wurde im Vergleich zu einer Kontrollgruppe (n = 54 Tiere) ermittelt, die nicht mit Creme behandelt wurden.

Aus der Tabelle 1 ist zu ersehen, daß die Wirkung bei der erfindungsgemäßen Creme erheblich schneller eintritt und die Wirkung insgesamt stärker ist als beim Handelsprodukt.

Tabelle 1

| | Inhibierungsgrad in % nach | | | | |
|---|---|---|---|---|---|
| | 1 h | 2 h | 3 h | 4 h | 5 h |
| Erfindungsgemäße Creme (n = 12 Tiere) | 76 | 81 | 81 | 73 | 66 |
| Handelsprodukt (n = 36 Tiere) | 29 | 53 | 53 | 50 | 44 |

Klinische Prüfung

In einer offen kontrollierten, randomisiert durchgeführten Studie wurde die erfindungsgemäße Creme und die im Handel befindliche Creme auf Wirksamkeit geprüft. Insgesamt wurden 14 Patienten mit Schulter-Arm-Syndrom behandelt, wobei in jeder Behandlungsgruppe nach Entrandomisierung 7 Patienten waren. Von den Cremes wurde jeweils 3-4 mal täglich ein 5-10 cm langer Strang auf das schmerzhafte Gebiet aufgetragen und großflächig in die Haut eingerieben. Beurteilt wurde der Ruhe-, Bewegungs-, und Druckschmerz sowie die Bewegungseinschränkung und die Griff-Festigkeit vor und nach 3 tägiger Behandlung. Wie aus der Tabelle 2 zu ersehen ist, wurde mit der erfindungsgemäßen Creme trotz der nur halben applizierten Menge an Ibuprofen ein günstigeres Therapieergebnis erzielt als mit der handelsüblichen Creme. Außerdem lehnten alle Patienten eine weitere Behandlung mit der handelsüblichen Creme ab, weil diese schlecht verreibbar ist und erst nach langem Reiben in die Haut einziehe. Dagegen läßt sich die erfindungsgemäße Creme gut und schnell einreiben und die Patienten verspüren einen raschen Wirkungseintritt.

Tabelle 2

| Anzahl der Patienten mit | | Erfindungsgemäße Creme (5 %ig) | | Handelsübliche Creme (10 %ig) | |
|---|---|---|---|---|---|
| | | vor der Therapie | nach 3 Tagen Therapie | vor der Therapie | nach 3 Tagen Therapie |
| Ruheschmerz* | n.v. | 0 | 0 | 1 | 1 |
| | l. | 0 | 5 | 1 | 4 |
| | m. | 6 | 2 | 5 | 2 |
| | s. | 1 | 0 | 0 | 0 |

# 0 087 062

Tabelle 2 (Fortsetzung)

| Anzahl der Fatienten mit | | Erfindungsgemäße Creme (5 %ig) | | Handelsübliche Creme (10 %ig) | |
|---|---|---|---|---|---|
| | | vor der Therapie | nach 3 Tagen Therapie | vor der Therapie | nach 3 Tagen Therapie |
| Bewegungs- schmerz* | n.v. | 0 | 0 | 0 | 0 |
| | l. | 0 | 3 | 0 | 1 |
| | m. | 2 | 4 | 2 | 5 |
| | s. | 5 | 0 | 5 | 1 |
| Druckschmerz* | n.v. | 0 | 0 | 0 | 1 |
| | l. | 1 | 3 | 3 | 4 |
| | m. | 5 | 4 | 4 | 2 |
| | s. | 1 | 0 | 0 | 0 |
| Bewegungseinschrän- kung* | n.v. | 0 | 2 | 0 | 2 |
| | l. | 3 | 5 | 5 | 5 |
| | m. | 4 | 0 | 2 | 0 |
| | s. | 0 | 0 | 0 | 0 |
| Grifffestigkeit** | n. | 0 | 4 | 0 | 3 |
| | l. | 6 | 3 | 6 | 4 |
| | s. | 1 | 0 | 1 | 0 |

Beurteilungsskala : * n.v. nicht vorhanden
l. leicht
m. mäßig
s. stark
** n. nicht eingeschränkt
l. leicht eingeschränkt
s. stark eingeschränkt

Ibuprofen-Serumspiegel

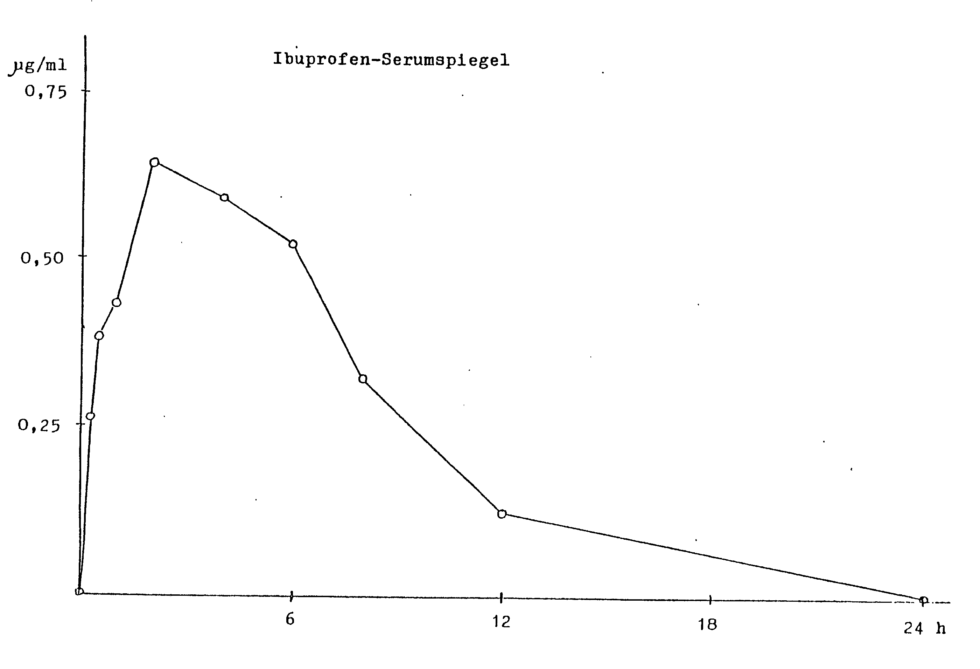

8 Probanden wurden jeweils ca. 6 g erfindungsgemäße Creme auf einem 20 mal 20 cm großem Hautareal eingerieben, nach bestimmten Zeiten Blut entnommen und der Ibuprofen-Gehalt bestimmt. Die Mittelwerte jeder Bestimmung sind in der Abbildung in Abhängigkeit von der Zeit dargestellt. Wie man sieht, wird innerhalb der ersten zwei Stunden Ibuprofen auf der erfindungsgemäßen Creme sehr schnell resorbiert.

**Patentansprüche**

1. Äußerlich anwendbares Arzneimittel, das eine wirksame Menge Ibuprofen in einem Träger enthält, dadurch gekennzeichnet, daß es 2 bis 12 Gewichtsteile Ibuprofen enthält, gelöst in einer Mischung aus 30 bis 50 Gewichtsteilen mittelkettiger Triglyceride, 4 bis 10 Gewichtsteilen Glycerinmonostearat-Polyoxyethylenstearat-Gemisch und 2 bis 10 Gewichtsteile Polyoxyethylen-Fettsäureester.

2. Arzneimittel gemäß Anspruch 1, dadurch gekennzeichnet, daß es in Form einer Creme vorliegt.

3. Arzneimittel gemäß Anspruch 2, dadurch gekennzeichnet, daß die wässrige Phase der Creme 2 bis 10 Gewichtsteile 1,2-Propandiol, 0,1 bis 1 Gewichtsteile 4-Hydroxybenzoesäuremethylester-Natrium und 0,1 bis 1 Gewichtsteil Xanthan-Gummi enthält.

4. Verfahren zur Herstellung eines äußerlich anwendbaren Arzneimittels, das eine wirksame Menge Ibuprofen in einem Träger enthält, dadurch gekennzeichnet, daß man 2 bis 12 Gewichtsteile Ibuprofen in einer Mischung aus 3 bis 50 Gewichtsteilen mittelkettiger Triglyceride, 4 bis 10 Gewichtsteile Glycerinmonostearat-Polyoxyäthylenstearat-Gemisch und 2 bis 10 Gewichtsteilen Polyoxyethylen-Fettsäureester unter Erwärmen löst und in an sich bekannter Weise zu einer Creme, einer Salbe oder einem Gel weiterverarbeitet.

5. Verfahren zur Herstellung einer Creme gemäß Anspruch 4, dadurch gekennzeichnet, daß man die erwärmte Lösung von Ibuprofen portionsweise unter Rühren mit einer wässrigen Lösung aus 2 bis 10 Gewichtsteilen 1,2-Propandiol, 0,1 bis 1 Gew.-% 4-Hydroxybenzoesäuremethylester-Natrium und 0,1 bis 1 Gewichtsteil Xanthan-Gummi versetzt und unter Rühren auf Raumtemperatur abkühlt.

## Claims

1. Medicament for external application containing an effective amount of Ibuprofen in a carrier, characterized in that it contains from 2 to 12 parts by weight of Ibuprofen dissolved in a mixture of from 30 to 50 parts by weight of medium-chain triglycerides, from 4 to 10 parts by weight of glycerol monostearatepolyoxyethylene stearate mixture and from 2 to 10 parts by weight of polyoxyethylene-fatty acid ester.

2. Medicament according to claim 1, characterized in that is in the form of a cream.

3. Medicament according to claim 2, characterized in that the aqueous phase of the cream contains from 2 to 10 parts by weight of 1.2-propanediol, from 0.1 to 1 parts by weight of 4-hydroxybenzoic acid methyl ester sodium and from 0.1 to 1 parts by weight of xanthane gum.

4. A process for preparing a medicament for external application containing an effective amount of Ibuprofen in a carrier, characterized in that from 2 to 12 parts by weight of Ibuprofen are dissolved with warming in a mixture of from 30 to 50 parts by weight of medium-chain triglycerides, from 4 to 10 parts by weight of glycerol monostearate-polyoxyethylene stearate mixture and from 2 to 10 parts by weight of polyoxyethylenefatty acid ester and are further processed in a per se known manner to form a cream, an ointment or a gel.

5. The process for preparing a cream according to claim 4, characterized in that an aqueous solution of from 2 to 10 parts by weight of 1.2-propanediol, from 0.1 to 1 parts by weight of 4-hydroxybenzoic acid methyl ester sodium and from 0.1 to 1 parts by weight of xanthane gum is added in portions to the warmed solution of Ibuprofen and the mixture is cooled to room temperature with stirring.

## Revendications

1. Médicament à usage externe, contenant une quantité efficace d'ibuprofène dans un véhicule et caractérisé par le fait qu'il contient 2 à 12 parties en poids d'ibuprofène dissous dans un mélange de 30 à 50 parties en poids de triglycérides à chaîne moyenne, 4 à 10 parties en poids de mélange monostéarate de glycérol-stéarate de polyoxyéthylène et 2 à 10 parties en poids d'ester d'acide gras de polyoxyéthylène.

2. Médicament selon la revendication 1, caractérisé par le fait qu'il se présente sous la forme d'une crème.

3. Médicament selon la revendication 2, caractérisé par le fait que la phase aqueuse de la crème contient 2 à 10 parties en poids de 1,2-propanediol, 0,1 à 1 partie en poids de 4-hydroxybenzoate de méthyle-sodium et 0,1 à 1 partie en poids de gomme xanthane.

4. Procédé de préparation d'un médicament à usage externe contenant une quantité efficace d'ibuprofène dans un véhicule, caractérisé par le fait que l'on dissout avec chauffage 2 à 12 parties en poids d'ibuprofène dans un mélange de 3 à 50 parties en poids de triglycérides à chaîne moyenne, 4 à 10 parties en poids de mélange monostéarate de glycérol/stéarate de polyoxyéthylène et 2 à 10 parties en poids d'ester d'acide gras de polyoxyéthylène et qu'on le transforme de manière connue en elle-même, en une crème, une pommade ou un gel.

5. Procédé de préparation d'une crème selon la revendication 4, caractérisé par le fait qu'à la solution chauffée d'ibuprofène, on ajoute par portions, en agitant, une solution aqueuse de 2 à 10 parties en poids de 1,2-propanediol, 0,1 à 1 % en poids de 4-hydroxybenzoate de méthyle-sodium et 0,1 à 1 partie en poids de gomme xanthane et que l'on refroidit jusqu'à la température ambiante en agitant.